(19) Europäisches Patentamt
European Patent Office
Office européen des brevets

(11) EP 0 658 547 B1

(12) **EUROPEAN PATENT SPECIFICATION**

(45) Date of publication and mention of the grant of the patent:
**11.03.1998 Bulletin 1998/11**

(51) Int Cl.6: **C07D 231/18**, C07D 231/38, A01N 43/56

(21) Application number: **94119428.4**

(22) Date of filing: **08.12.1994**

(54) **N-pyrazolyl carbamate derivative, agricultural/horticultural fungicide containing the same as active ingredient and production intermediate thereof**

N-Pyrazolylcarbamatderivate und Fungizide für Landwirtschaft und Gartenbau, die diese als aktive Bestandteile erhalten, Produktion und Zwischenprodukte

Dérivés de N-pyrazolyl carbamates et fongicides agricoles et horticoles le contenant comme ingrédient actif, procédé de préparation et produits intermédiaires

(84) Designated Contracting States:
**BE CH DE DK ES FR GB IT LI NL**

(30) Priority: **14.12.1993 JP 313520/93**

(43) Date of publication of application:
**21.06.1995 Bulletin 1995/25**

(73) Proprietor: **MITSUBISHI CHEMICAL CORPORATION**
**Chiyoda-ku, Tokyo (JP)**

(72) Inventors:
- **Oda, Masatsugu,**
**Mitsubishi Chemical Corporation**
**Aoba-ku, Yokohama-shi, Kanagawa-ken (JP)**
- **Katsurada, Manabu,**
**Mitsubishi Chemical Corporation**
**Aoba-ku, Yokohama-shi, Kanagawa-ken (JP)**
- **Tomita, Hirofumi,**
**Mitsubishi Chemical Corporation**
**Aoba-ku, Yokohama-shi, Kanagawa-ken (JP)**

(74) Representative:
**TER MEER STEINMEISTER & PARTNER GbR**
**Mauerkircherstrasse 45**
**81679 München (DE)**

(56) References cited:
**EP-A- 0 129 830**
**EP-A- 0 433 899**
**EP-A- 0 483 851**
**EP-A- 0 571 326**

- **PATENT ABSTRACTS OF JAPAN vol. 17, no. 644 (C-1134) (6273) 30 November 1993 & JP-A-05 201 980 (MITSUBISHI KASEI CORPORATION) 10 August 1993**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a novel N-pyrazolyl carbamate derivative and an agricultural/horticultural fungicide containing the same as an active ingredient.

### BACKGROUND OF THE INVENTION

It has hitherto been known that a certain N-pyrazolyl carbamate derivative has a biological activity such as herbicidal effect. For example, in EP129830, there is described a compound of the formula:

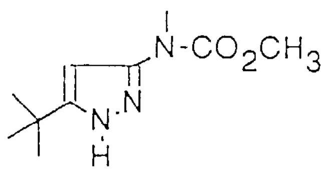

as a compound having a herbicidal activity.

In DE3423582, there is also described a compound of the formula:

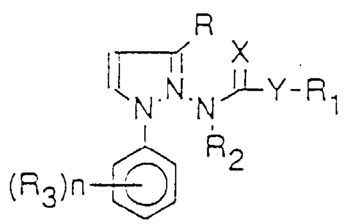

wherein R is a cyano group or an alkoxycarbonyl group: $R_1$ is an alkyl group or an aryl group; $R_2$ is a hydrogen atom or a group of $-C(X)YR_1$; and X and Y indicate an oxygen atom or a sulfur atom, as a compound having a herbicidal activity.

EP-A-571 326 describes pyrazolyl acetic acid derivatives and their use for the control of phytopathogens.

EP-A-483 851 discloses pyrazolyl acrylic acid derivatives and agricultural/horticultural fungicides containing said derivatives.

JP-A-5-201 980 discloses methoxyiminoacetic acid derivatives and agricultural/horticultural bactericides containing said derivatives.

However, these compounds are not necessarily suitable as an agricultural/horticultural fungicide at present.

### OBJECTS OF THE INVENTION

The present inventors have intensively studied about the N-pyrazolyl carbamate derivative. As a result, it has been found that a N-pyrazolyl carbamate derivative having a certain structure has not only excellent fungicidal activity but also extremely excellent systemicity in plants. The present invention is based on such finding.

Thus, one object of the present invent ion is to provide a N-pyrazolyl carbamate derivative which has an excellent fungicidal activity.

Another object of the present invention is to provide an agricultural/horticultural fungicide containing the N-pyrazolyl carbamate derivative as an active ingredient, which has excellent preventive effects on various phytopathogenic fungi.

These objects as well as other objects and advantages of the present invention will become apparent to those skilled in the art from the following description.

### SUMMARY OF THE INVENTION

The present invention provides a N-pyrazolyl carbamate derivative represented by the general formula (I):

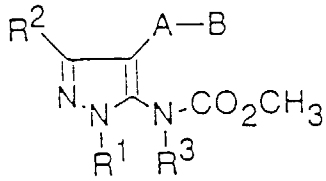

(I)

wherein $R^1$ and $R^2$ independently indicate a hydrogen atom or a $C_1$-$C_4$ alkyl group; $R^3$ is a hydrogen atom, a $C_1$-$C_4$ alkyl group, a $C_2$-$C_5$ alkynyl group, a $C_2$-$C_4$ alkylthioalkyl group or a $C_2$-$C_4$ alkoxyalkyl group; A is -O-, -C(O)-, $-OCH_2-$, $-CH_2O-$, $-CH_2S-$, -C≡C-, -CH=CH-, $-CH_2CH_2-$, $-CH_2ON=C(R^4)-$, or $-CH=NOC(R^4)(R^5)-$ (wherein $R^4$ and $R^5$ independently indicate a hydrogen atom or a $C_1$-$C_4$ alkyl group); and B is a hydrogen atom, an aryl group selected from phenyl and naphthyl groups or a heterocyclic group selected from pyridyl, pyrimidyl, furanyl and benzothiazolyl groups, said aryl and heterocyclic groups being optionally substituted with a substituent selected from the group consisting of a cyano group; a nitro group; a halogen atom; a $C_1$-$C_4$ alkyl group; a $C_1$-$C_4$ haloalkyl group; a $C_1$-$C_6$ alkoxy group optionally substituted with a halogen atom or a $C_3$-$C_6$ cycloalkyl group; a $C_1$-$C_6$ alkylthio group; a $C_2$-$C_6$ alkenyloxy group optionally substituted with a halogen atom; a $C_2$-$C_6$ alkynyloxy group; and a phenoxy, benzyloxy or pyridyloxy group optionally substituted with a cyano group, nitro group, a halogen atom, a $C_1$-$C_4$ alkyl group, a $C_1$-$C_4$ haloalkyl group, a $C_1$-$C_6$ alkoxy group optionally substituted with a halogen atom or a $C_3$-$C_6$ cycloalkyl group, $C_1$-$C_6$ alkylthio group, $C_2$-$C_6$ alkenyloxy group which may be substituted with a halogen atom, or a $C_2$-$C_6$ alkynyloxy group.

The present invention also provides an agricultural/ horticultural fungicide containing the same as an active ingredient.

DETAILED DESCRIPTION OF THE INVENTION

The present invention will be explained in detail below.

The N-pyrazolyl carbamate derivative of the present invention is represented by the above formula (I). In the above formula (I), $R^1$ and $R^2$ independently indicate a hydrogen atom; or a $C_1$-$C_4$ alkyl group such as methyl group, ethyl group, n-propyl group, iso-propyl group, n-butyl group, sec-butyl group, etc. They indicate preferably hydrogen atom, methyl group or ethyl group, and more preferably, methyl group.

$R^3$ is a hydrogen atom; a $C_1$-$C_4$ alkyl group such as methyl group, ethyl group, n-propyl group, iso-propyl group, n-butyl group, sec-butyl group, etc.;a $C_2$-$C_5$ alkynyl group such as ethynyl group, propargyl group, butynyl group, pentynyl group, etc.; a $C_2$-$C_4$ alkylthioalkyl group such as methylthiomethyl group, ethylthiomethyl group, etc.; or a $C_2$-$C_4$ alkoxyalkyl group such as methoxymethyl group, ethoxymethyl group, methoxyethyl group, ethoxyethyl group, etc. It is preferably hydrogen atom, $C_2$-$C_5$ alkynyl group or $C_2$-$C_4$ alkoxyalkyl group.

A is -O-, -C(O)-, $-OCH_2-$, $-CH_2O-$, $-CH_2S-$, -C≡C-, -CH=CH-, $-CH_2CH_2-$, $-CH_2ON=C(R^4)-$ or $-CH=NOC(R^4)(R^5)-$. It is preferably -O-, $-OCH_2-$, $-CH_2O-$, $-CH_2S-$, -C≡C-, -CH=CH-, $-CH_2ON=C(R^4)-$ or $-CH=NOC(R^4)(R^5)-$, and more preferably, $-OCH_2-$, $-CH_2O-$, $-CH_2S-$, -C≡C-, $-CH_2ON=C(R^4)-$ or $-CH=NOC(R^4)(R^5)-$. $R^4$ and $R^5$ independently indicate a hydrogen atom; or a $C_1$-$C_4$ alkyl group such as methyl group, ethyl group, n-propyl group, iso-propyl group, n-butyl group, sec-butyl group, etc. They indicate preferably hydrogen atom or $C_1$-$C_2$ alkyl group. More preferably, $R^4$ is a hydrogen or a methyl group and $R^5$ is a hydrogen atom.

B is a hydrogen atom; an aryl group selected from a phenyl group, and a naphthyl group; or a heterocyclic group selected from pyridyl group, pirimidyl group, furanyl group and, benzothiazolyl group. It is preferably an optionally substituted phenyl group , naphthyl group or pyridyl group, and more preferably, optionally substituted phenyl group.

The aryl group and heterocyclic group may be substituted with a substituent selected from a group consisting of the following substituents: a cyano group; a nitro group; a halogen atom such as fluorine atom, chlorine atom, bromine atom, etc.; a $C_1$-$C_4$ alkyl group such as methyl group, ethyl group, n-propyl group, iso-propyl group, n-butyl group, sec-butyl group, etc.; a $C_1$-$C_4$ haloalkyl group such as trifluoromethyl group, difluoromethyl group, trichloromethyl group, dichlorodifluoroethyl group etc.; a $C_1$-$C_6$ alkoxy group such as methoxy group, ethoxy group, isopropoxy group, n-butoxy group, etc. which may be substituted with a halogen atom or a $C_3$-$C_6$ cycloalkyl group; a $C_1$-$C_6$ alkylthio group such as methylthio group, ethylthio group, iso-propylthio group, n-butylthio group, etc.; a $C_2$-$C_6$ alkenyloxy group such as propenyloxy group, etc. which may be substituted with a halogen atom; a $C_2$-$C_6$ alkynyloxy group such as propargyloxy group, etc.; and a phenoxy group, a benzyloxy group, or a pyridyloxy group which may be substituted by the just mentioned cyano group, nitro group, halogen atom, $C_1$-$C_4$ alkyl group, $C_1$-$C_4$ haloalkyl group, $C_1$-$C_6$ alkoxy group, $C_1$-$C_6$ alkylthio group, $C_1$-$C_6$ alkenyloxy group, or $C_1$-$C_6$ alkynyloxy group.

Further, adjacent two substituents may bond together with an aryl group or a heterocyclic group to form a fused ring. The number of the substituent is 0 to 5, preferably 0 to 3. When containing a plurality of substituents, the substituents may be the same or different. Preferable examples of the substituent include halogen atom, $C_1$-$C_4$ alkyl group,

$C_1$-$C_4$ haloalkyl group, $C_1$-$C_6$ alkoxy group which may be substituted with a halogen atom, phenoxy group and the like. More preferable examples of the substituent include halogen atom; $C_1$-$C_4$ alkyl group; $C_1$-$C_4$ alkoxy group which may be substituted with a halogen atom, preferably with a fluorine atom; trifluoromethyl group; phenoxy group and the like.

All compounds of the present invention are novel and they can be produced according to the following reaction scheme 1, 2 or 3.

(Reaction scheme 1)

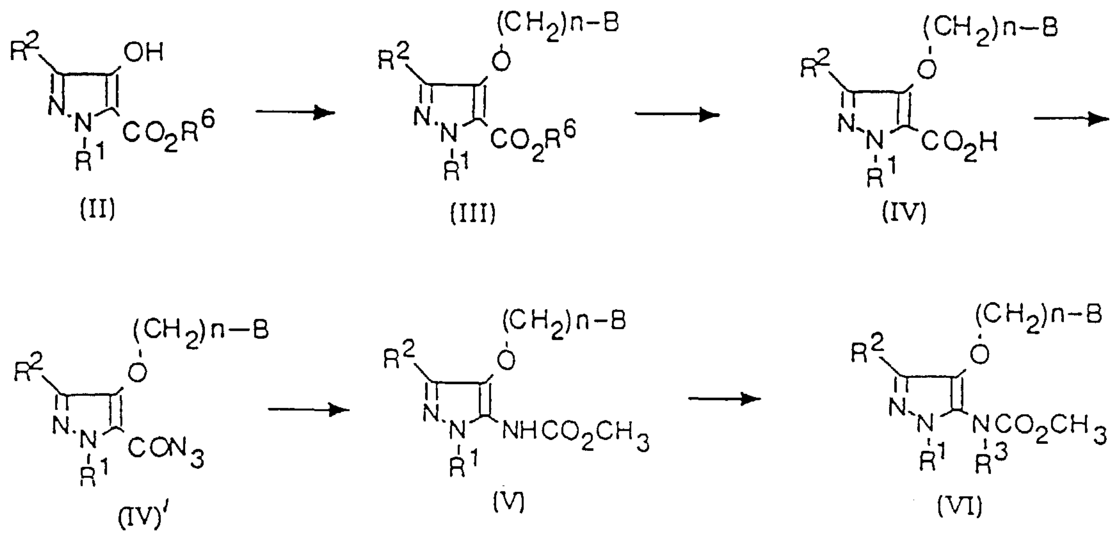

(II) (III) (IV) (IV)' (V) (VI)

In the above reaction scheme, $R^1$, $R^2$, $R^3$ and B are as defined in the above general formula (I); $R^6$ is a $C_1$-$C_4$ alkyl group; and n is 0 or 1.

(Reaction scheme 2)

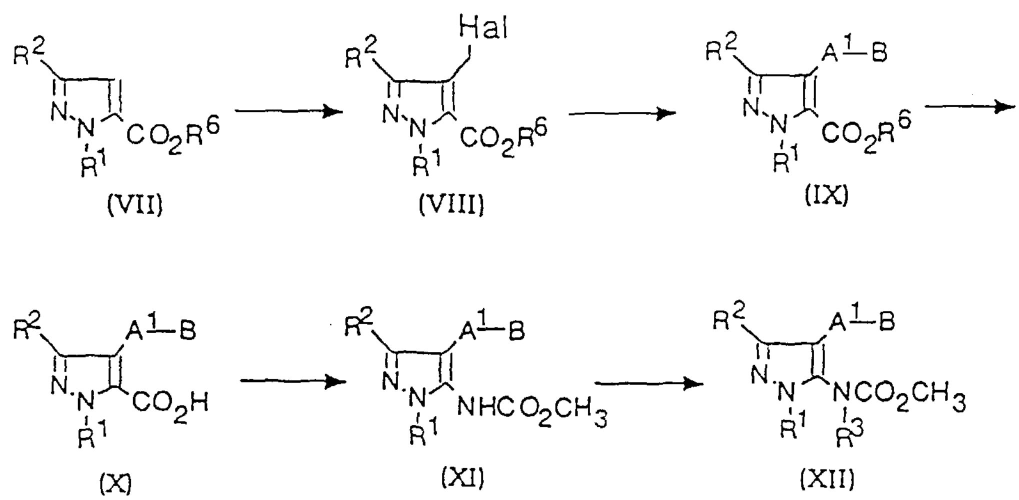

(VII) (VIII) (IX) (X) (XI) (XII)

(Reaction scheme 3)

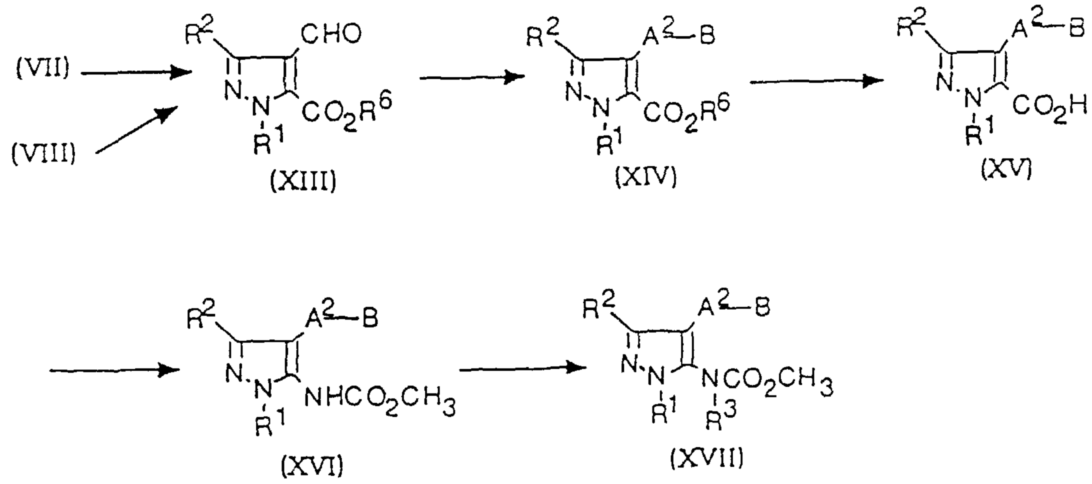

(VII) (VIII) (XIII) (XIV) (XV) (XVI) (XVII)

In the reaction schemes 2 and 3, $R^1$, $R^2$, $R^3$ and B are as defined in the above general formula (I); Hal is a chlorine atom or a bromine atom; $R^6$ is a $C_1$-$C_4$ alkyl group; $A^1$ is $-CH_2O-$, $-CH_2S-$ or $-CH_2ON=C(R^4)-$ and $A^2$ is $-CH=NOC(R^4)(R^5)-$.

The above reaction scheme 1 shows a method comprising reacting a 4-hydroxy-5-alkoxycarbonyl pyrazole derivative (II) with a halide $-(CH_2)_nB$ in the presence of a base to give a corresponding ether derivative (III); converting a carboxylic acid derivative (IV), obtained by hydrolyzing an ester group with a suitable base, into a carboxylic acid halide with a halogenating agent; and then subjecting an azide derivative (IV)', obtained by treating sodium azide, to the Curtius rearrangement reaction in methanol to give a carbamate derivative (V). The carbamate derivative (V) can be reacted with a corresponding halide in the presence of a base to give a compound (IV). Further, 4-hydroxy-5-alkoxycarbonyl pyrazole (II) as a starting material can be produced by a method described in Japanese Patent Application No. 4-217668 or a similar method.

Examples of the base used in the reaction from (II) to (III) and the reaction from (V) to (VI) include alkali metal hydrides such as sodium hydride, etc.; alkali metal alcolates such as sodium methylate, etc.; alkali metal carbonates such as potassium carbonate, etc.; alkali metal hydroxides such as potassium hydroxide, etc. Preferable examples include alkali metal hydrides and alkali metal carbonates.

Examples of the base used in the reaction from (III) to (IV) include alkali metal hydroxides such as sodium hydroxide, potassium hydroxide, etc.

Examples of the halogenating agent used in the reaction from (IV) to carboxylic acid halide include thionyl chloride, phosgene or phosphorous oxychloride. In this reaction, there can be used tertiary amines such as N-methylmorpholine, triethylamine, etc., aromatic bases such as pyridine, picoline, etc. in an inert solvent. The following solvent may be used alone in the azidation reaction, or the reaction may be carried out in two kinds of solvents in the presence or absence of a normal phase transfer catalyst.

Examples of the solvent used in these reactions include water; alcohols such as methanol, ethanol, etc.; ethers such as diethyl ether, tetrahydrofuran, etc.; polar solvents such as dimethylformamide, dimethyl sulfoxide, etc.; aromatic hydrocarbons such as toluene, chlorobenzene, etc.; alkyl halides such as methylene chloride, dichloroethane, etc.

The reaction scheme 2 shows a method comprising subjecting a 5-alkoxycarbonyl pyrazole derivative (VII) to chloromethylation or bromomethylation, converting into an ether, thioether or iminooxy derivative (IX) and then preparing carbamate derivatives (XI) and (XII) according to the same manner as that described in the reaction scheme 1.

The chloromethylation or bromomethylation reaction from (VII) to (VIII) can be carried out according to a method described in Org. Synth., III, 195 or a similar method. The derivative (VIII) can be reacted with B-OH, B-SH or an oxime derivative in an inert solvent {e.g., alcohols such as methanol, ethanol, etc.; ethers such as diethyl ether, tetrahydrofuran, etc.; polar solvents such as dimethylformamide (DMF), dimethyl sulfoxide, etc.; aromatic hydrocarbons such as toluene, chlorobenzene, etc.} in the presence of a suitable base (e.g., alkali metal hydrides such as sodium hydride, etc.; alkali metal alcoholates such as sodium methylate, etc.; alkali metal carbonates such as potassium carbonate, etc.; alkali metal hydroxides such as potassium hydroxide, etc.) to give an ether, thioether or iminooxy derivative (IX).

The reaction scheme 3 shows a method comprising subjecting a 5-alkoxycarbonyl pyrazole derivative (VII) to the Vilsmeirer reaction or subjecting a chloromethyl or bromomethyl derivative (VIII) to the oxidation reaction to give an

aldehyde derivative (XIII) and, after subjecting to imination, preparing carbamate derivatives (XVI) and (XVII).

The Vilsmeirer reaction of the compound (VII) itself is a known method and can be carried out according to a method described in Org. Synth., III, 98 (1955) or Zh. Org. Khim., 9, 815 (1973) or a similar method. The oxidation reaction of the compound (VIII) can be carried out by a method of treating N-oxide of tertiary amine (e.g., N-methylmorpholine or triethylamine) in an inert solvent (e.g., methylene chloride or acetonitrile).

The resulting aldehyde derivative (XIII) can be reacted with a corresponding oxyamine derivative in an inert solvent (e.g., alcohols such as methanol, ethanol, etc.; ethers such as diethyl ether, tetrahydrofuran, etc.; polar solvents such as dimethylformamide, dimethyl sulfoxide, etc.; aromatic hydrocarbons such as toluene, chlorobenzene, etc.) to give an imino derivative (XIV).

All reactions described in reaction schemes 1 to 3 are carried out at a temperature within a range from -20°C to the boiling point of the solvent to be used.

The compounds of the present invention thus obtained are each novel one having an excellent fungicidal activity. They exert excellent preventive effects on various phytopathogenic fungi, which makes them useful as an agricultural/horticultural fungicide.

For example, the compound of the present invention has a high activity against rice blast (Pyricularia oryzae), rice sheath blight (Rhizoctonia solani), wheats powdery mildow (Erysiphe graminis f. sp. tritici), barley powdery mildow (E. graminis f. sp. hodei), various leaf rusts of wheat and barley (e.g., Puccinia recondita), gray mold of vegetables and fruit trees (Botrytis cinerea), late blight of various crops (Phytophthora infestans) and the like. Further, they have prolonged residual activity and excellent systemicity in plants, which makes them highly useful as an agricultural/horticultural fungicide.

When using the compound of the present invention as an agricultural/horticultural fungicide, the compound may be used as it is. It is preferable to formulate said compound into, for example, emulsifiable concentrates, wettable powders, dust or granules by blending with adjuvants in a conventional manner to thereby ensure the effective dispersion of the active ingredient at the application.

When using the agricultural/horticultural fungicide of the present invention in the form of emulsifiable concentrate, a raw material obtained by mixing 10 to 80 parts (preferably 10 to 70 parts) of the compound of the present invention, 10 to 90 parts (preferably 20 to 80 parts) of a solvent and 3 to 20 parts (preferably 5 to 15 parts) of a surfactant is diluted with water to a predetermined concentration and the resulting chemical solution is applied by a method such as spraying.

When using the fungicide in the form of wettable powder, a raw material obtained by mixing 5 to 80 parts (preferably 10 to 70 parts) of the compound of the present invention, 10 to 90 parts (preferably 20 to 80 parts) of an extender and 1 to 20 parts (preferably 3 to 15 parts) of a surfactant is diluted with water to a predetermined concentration according to the same manner as the case of emulsifiable concentrate.

When using the fungicide in the form of dust, a raw material obtained by mixing 0.1 to 10 parts (preferably 1 to 5 parts) of the compound of the present invention and 90 to 99.9 parts (preferably 95 to 99 parts) of an extender such as kaolin, bentonite, talc , etc. is used as it is.

The agricultural/horticultural fungicide of the present invention can also be used after mixing with other active ingredients which do not inhibit the fungicidal effect of the active ingredient of the present invention such as fungicides, insecticides, acarcides and the like.

The agricultural/horticultural fungicide of the present invention can be suitably applied for both foliar application and submerged application. In case of foliar application, emulsifiable concentrates or wettable powders are normally diluted with water to the concentration (concentration of active ingredient) of 10 to 1000 ppm and the resulting solution may be applied in an amount of 10 to 500 liters per 10 ares.

Among the compounds represented by the above formulas (VIII) and (XIII), a compound wherein $R^1$ and $R^2$ indicate an $C_1$-$C_4$ alkyl group is novel and is useful as a production intermediate of the compound of the general formula (I).

As described above, all compounds of the present invention are novel compounds and they have an excellent fungicidal activity. Since the compound of the present invention has an excellent control effect on various phytopathogenic fungi, it is useful as an agricultural/horticultural fungicide.

## Examples

The following Examples further illustrate the present invention in detail but are not to be construed as to limiting the scope thereof.

Example 1

Synthesis of methyl N-{1,3-dimethyl-4-(2,5-dimethylbenzyloxy)pyrazole-5-yl}-N-propargyl-carbamate (compound No. 50 in Table 1)

A DMF solution (10 ml) of methyl 1,3-dimethyl-4-hydroxypyrazole-5-ylcarboxylate(1 g), 2,5-dimethylbenzyl chloride (0.95 g) and $K_2CO_3$ (0.98 g) were heated with stirring for 2 hours. 30 ml of ethyl acetate was added, and the mixture was washed in turn with water and brine and then dried over anhydrous sodium sulfate. The solvent was distilled off to give a crude benzyl ether derivative (1.5 g).

An aqueous NaOH solution (NaOH: 0.25 g, $H_2O$: 5 ml) was added to an ethanol (5 ml) solution of the benzyl ether derivative (1.5 g) and the mixture was heated under reflux condition for two hours. After cooling, it was neutralized by adding concentrated HCl (0.65 ml), followed by extracting with 30 ml of ethyl acetate. Then, the organic layer was washed in turn with water and brine and dried over anhydrous sodium sulfate. The solvent was distilled off and the resulting residue was recrystallized from a mixed solvent of ethyl acetate/hexane to give a carboxylic acid derivative (1.4 g).

To 15 ml of a methylene chloride solution of the carboxylic acid derivative (1.4 g), pyridine (0.7 g) and thionyl chloride (0.77 g) were added in turn under water cooling. After stirring for 2 hours, an aqueous sodium azide solution ($NaN_3$: 0.5 g, $H_2O$: 2 ml) and $(n\text{-}C_4H_9)_4NCl$ (0.05 g) were added. After stirring for 3 hours, 30 ml of methylene chloride was added, and the organic layer was washed in turn with water and brine and then dried over anhydrous sodium sulfate. The solvent was distilled off to give a crude azide derivative (1.5 g).

To the crude azide derivative (1.5 g), 20 ml of methanol was added and the mixture was heated under reflux condition for 4 hours. After the solvent was distilled off, the residue was chromatographed ($SiO_2$: 50 g, hexane/ethyl acetate = 1/1) to give a 1.3 g of methyl N-{1,3-dimethyl-4-(2,5-dimethylbenzyloxy)pyrazole-5-yl} carbamate (compound No. 48 in Table 1).

To 5 ml of a DMF solution of the carbamate derivative (0.8 g, compound No. 48 in Table 1), 60% NaH (0.11 g) was added and the mixture was stirred for 20 minutes. Then, propargyl bromide (1 g) was added, followed by stirring for one hour. After 30 ml of ethyl acetate was added, the mixture was washed in turn with water and brine and dried over anhydrous sodium sulfate. The solvent was distilled off, and then the residue was chromatographed ($SiO_2$: 50 g, hexane/ethyl acetate = 1/1) to give 0.8 g of a titled compound.

Example 2

Synthesis of methyl N-[1,3-dimethyl-4-{(α-methyl-4-trifluoromethyl-benzylidene)aminooxymethyl pyrazole-5-yl}]-N-propargyl-carbamate (compound No. 169 in Table 1)

To a solution of ethyl 1,3-dimethylpyrazole-5-ylcarboxylate (16.3 g), phosphoric acid (11 ml), concentrated hydrochloric (27 ml) and acetic acid (50 ml), paraformaldehyde (6 g) was added and the mixture was heated at 100°C with stirring for 5 hours. After cooling, the reaction mixture was poured into ice and neutralized with $Na_2CO_3$, and extracted with 200 ml of ethyl acetate. The extract was washed in turn with water and brine and dried over anhydrous sodium sulfate. The solvent was distilled off and the residue was chromatographed ($SiO_2$: 150 g, hexane/ethyl acetate = 4/1) to give 8 g of ethyl 4-chloromethyl-1,3-dimethylpyrazole-5-ylcarboxylate.

$^1$H-NMR ($CDCl_3$); 1.42 (3H, t), 2.30 (3H, s), 4.12 (3H, s), 4.42 (2H, q), 4.75 (2H, s)

60% NaH (0.2 g) was added to a DMF solution (5 ml) of 4-trifluoromethylacetophenone oxime (1 g) and the mixture was stirred for 20 minutes. Then, ethyl 4-chloromethyl-1,3-dimethylpyrazole-5-ylcarboxylate (1 g) was added and the mixture was stirred and allowed to stand overnight. After 30 ml of ethyl acetate was added, the organic layer was washed in turn with water and brine and then dried over anhydrous sodium sulfate. The solvent was distilled off to give a crude oxime ether derivative (1.8 g).

To 10 ml of an ethanol solution of the oxime ether derivative (1.8 g), an aqueous NaOH solution (NaOH: 0.24 g, $H_2O$: 5 ml) was added and the mixture was heated under reflux condition for 2 hours. After cooling, the reaction mixture was neutralized by adding concentrated HCl (0.6 ml). After 30 ml of ethyl acetate was added, the organic layer was washed in turn with water and saturated saline and then dried over anhydrous sodium sulfate. The solvent was distilled off and the resulting residue was recrystallized from a mixed solvent of ethyl acetate/hexane to give a carboxylic acid derivative (1.6 g).

To 15 ml of a methylene chloride solution of the carboxylic acid derivative (1.6 g), pyridine (0.6 g) and thionyl chloride (0.6 g) were added in turn under water cooling. After stirring for 2 hours, an aqueous sodium azide solution ($NaN_3$: 0.4 g, $H_2O$: 2 ml) and $(n\text{-}C_4H_9)$,NCl (0.05 g) were added. After stirring for 3 hours, 30 ml of methylene chloride was added, and the organic layer was washed in turn with water and brine and then dried over anhydrous sodium

sulfate. The solvent was distilled off to give a crude azide derivative (1.6 g).

To the crude azide derivative (1.6 g), 20 ml of methanol was added and the mixture was heated under reflux condition for 4 hours. After the solvent was distilled off, the residue was chromatographed ($SiO_2$: 50 g, hexane/ethyl acetate = 1/1) to give a 1.4 g of methyl N-[1,3-dimethyl-4-{(α-methyl-4-trifluoromethylbenzylidene)aminooxymethyl pyrazole-5-yl}] carbamate (compound No. 167 in Table 1).

To 5 ml of a DMF solution of the carbamate derivative (1.4 g, compound No. 167 in Table 1), 60% NaH (0.16 g) was added and the mixture was stirred for 20 minutes. Then, propargyl bromide (1 g) was added and the mixture was stirred for one hour. After 30 ml of ethyl acetate was added, the mixture was washed in turn with water and brine and then dried over anhydrous sodium sulfate. After the solvent was distilled off, the residue was chromatographed ($SiO_2$: 50 g, hexane/ethyl acetate = 1/1) to give 1.2 g of a titled compound.

Example 3

Synthesis of N-{1,3-dimethyl-4-(α-methyl-3-trifluoromethyl-benzyloxyiminomethyl)pyrazole-5-yl} carbamate (compound No. 194 in Table 1)

To a mixture of ethyl 1,3-dimethylpyrazole-5-ylcarboxylate (20.0 g) and a 30% hydrogen bromide-acetic acid solution (60 ml), paraformaldehyde (7.2 g) was added and the mixture was stirred at 80 - 90°C for 2 hours and a half. The reaction mixture was poured into ice water and the solution was neutralized with sodium acetate trihydrate (41 g). After extracting with ethyl acetate, the extract was washed in turn with an aqueous saturated sodium bicarbonate solution and brine and then dried over anhydrous sodium sulfate. After the solvent was distilled off, the residue was chromatographed ($SiO_2$: 200 g, hexane/ethyl acetate = 4/1) to give 26.8 g of ethyl 4-bromomethyl-1,3-dimethylpyrazole-5-ylcarboxylate (yield: 86.3%).

$^1$H-NMR ($CDCl_3$); 1.45 (3H, t), 2.28 (3H, s), 4.09 (3H, s), 4.42 (2H, q), 4.65 (2H, s),
$^{13}$C-NMR ($CDCl_3$); 11.3, 14.1, 23.2, 39.8, 61.4, 120.3, 130.3, 147.1, 159.7

To 20 ml of an acetonitrile solution of ethyl 4-bromomethyl-1,3-dimethylpyrazole-5-ylcarboxylate (2.9 g, 11.1 mmol) and molecular sieves 4A (3 g), N-methylmorpholine-N-oxide (2.6 g, 22.2 mmol) was added under water cooling, followed by stirring at room temperature for 48 hours. The mixed solution was filtered with celite and the filtrate was concentrated. To the resulting residue, ethyl acetate was added and the mixture was washed in turn with water and brine and then dried over anhydrous sodium sulfate. After the solvent was distilled off, the resulting residue was recrystallized from hexane/ethyl acetate to give 1.7 g of ethyl 1,3-dimethyl-4-formylpyrazole-5-ylcarboxylate (yield: 78%).

$^1$H-NMR ($CDCl_3$); 1.44 (3H, t), 2.49 (3H, s), 4.14 (3H, s), 4.47 (2H, q), 10.39 (1H, s)

To 15 ml of an ethanol solution of ethyl 1,3-dimethyl-4-formylpyrazole-5-ylcarboxylate (1.5 g, 7.65 mmol), α-methyl-3-trifluoromethylbenzyloxyamine (1.6 g, 7.80 mmol) was added and the mixture was stirred at room temperature for 12 hours. The reaction solution was concentrated and the residue was chromatographed ($SiO_2$: 40 g, hexane/ethyl acetate = 4/1) to give 2.5 g of ethyl {1,3-dimethyl-4-(α-methyl-3-trifluoromethyl-benzyloxyiminomethyl)pyrazole-5-yl} carboxylate (yield: 85.3%).

$^1$H-NMR ($CDCl_3$); 1.42 (3H, t), 1.63 (3H, d), 2.23 (3H, s), 4.07 (3H, s), 4.40 (2H, q), 4.73 (1H, q), 7.4-7.65 (4H, m), 8.56 (1H, s)

According to the same manner as that described in Examples 1 and 2, the resulting ethyl carboxylate derivative (2.5 g) was subjected in turn to hydrolysis, azidation and Curtius rearrangement reactions to give the titled compound (1.9 g).

According to the same manner as that described above except for an alteration in the starting material, a compound in Table 1 can be synthesized.

Table 1

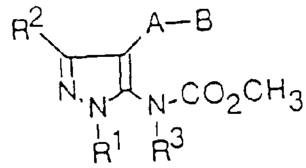

| Compound No. | R1 | R2 | R3 | A | B | Physical properties |
|---|---|---|---|---|---|---|
| 1 | CH3 | CH3 | H | O | H | White crystal |
| 2 | CH3 | CH3 | H | O | 5-CF3-pyridin-2-yl | White crystal |
| 3 | CH3 | CH3 | CH2OCH3 | O | 5-CF3-pyridin-2-yl | Viscous liquid |
| 4 | CH3 | CH3 | propargyl | O | 5-CF3-pyridin-2-yl | Viscous liquid |
| 5 | CH3 | CH3 | CH2SCH3 | O | 5-CF3-pyridin-2-yl | Viscous liquid |
| 6 | CH3 | CH3 | CH2OCH3 | O | 4-CF3-pyridin-2-yl | Viscous liquid |
| 7. | CH3 | CH3 | propargyl | O | 4-CF3-pyridin-2-yl - | Viscous liquid |
| 8 | CH3 | CH3 | CH2OCH3 | O | 3-CF3-pyridin-2-yl | Viscous liquid |
| 9 | CH3 | CH3 | propargyl | O | 3-CF3-pyridin-2-yl | Viscous liquid |
| 10 | CH3 | CH3 | CH2OCH3 | O | 4-CF3-6-Cl-pyridin-2-yl | Viscous liquid |
| 11 | CH3 | CH3 | propargyl | O | 4-CF3-6-Cl-pyridin-2-yl | Viscous liquid |
| 12 | CH3 | CH3 | CH2OCH3 | O | 4-Cl-pyridin-2-yl | Viscous liquid |
| 13 | CH3 | CH3 | C2H5 | O | 4-Cl-pyridin-2-yl | Viscous liquid |
| 14 | CH3 | CH3 | propargyl | O | 4,6-Cl2-pyridin-2-yl | Viscous liquid |
| 15 | CH3 | CH3 | propargyl | O | 5-Cl-2-NO2-phenyl | Viscous liquid |
| 16 | CH3 | CH3 | C2H5 | O | 3-Cl-phenyl | Viscous liquid |
| 17 | CH3 | CH3 | propargyl | O | 3-PhO-phenyl | Viscous liquid |
| 18 | CH3 | CH3 | propargyl | O | 3-Cl-4-NO2-phenyl | Viscous liquid |
| 19 | CH3 | CH3 | propargyl | O | 6-(2'-CN-PhO)pyrimidin-4-yl | Viscous liquid |
| 20 | CH3 | CH3 | propargyl | O | 4-(2'-CN-PhO)pyrimidin-2-yl | Viscous liquid |
| 21 | CH3 | CH3 | H | OCH2 | Phenyl | White crystal |
| 22 | CH3 | CH3 | CH2OCH3 | OCH2 | Phenyl | Viscous liquid |
| 23 | CH3 | CH3 | propargyl | OCH2 | Phenyl | Viscous liquid |
| 24 | CH3 | CH3 | CH2SCH3 | OCH2 | Phenyl | Viscous liquid |
| 25 | CH3 | CH3 | H | OCH2 | 3-Cl-phenyl | White crystal |

Table 1 (Continued)

| | | | | | | |
|---|---|---|---|---|---|---|
| 26 | CH3 | CH3 | C2H5 | OCH2 | 3-Cl-phenyl | Viscous liquid |
| 27 | CH3 | CH3 | CH2OCH3 | OCH2 | 3-Cl-phenyl | Viscous liquid |
| 28 | CH3 | CH3 | CH2SCH3 | OCH2 | 3-Cl-phenyl | Viscous liquid |
| 29 | CH3 | CH3 | propargyl | OCH2 | 3-Cl-phenyl | Viscous liquid |
| 30 | CH3 | CH3 | propargyl | OCH2 | 2-Cl-phenyl | Viscous liquid |
| 31 | CH3 | CH3 | CH2OCH3 | OCH2 | 2-Cl-phenyl | Viscous liquid |
| 32 | CH3 | CH3 | propargyl | OCH2 | 4-Cl-phenyl | Viscous liquid |
| 33 | CH3 | CH3 | CH2OCH3 | OCH2 | 4-Cl-phenyl | Viscous liquid |
| 34 | CH3 | CH3 | propargyl | OCH2 | 2,4-Cl2-phenyl | Viscous liquid |
| 35 | CH3 | CH3 | CH2OCH3 | OCH2 | 2,4-Cl2-phenyl | Viscous liquid |
| 36 | CH3 | CH3 | H | OCH2 | 2,5-Cl2-phenyl | White crystal |
| 37 | CH3 | CH3 | C2H5 | OCH2 | 2,5-Cl2-phenyl | Viscous liquid |
| 38 | CH3 | CH3 | CH2OCH3 | OCH2 | 2,5-Cl2-phenyl | Viscous liquid |
| 39 | CH3 | CH3 | CH2SCH3 | OCH2 | 2,5-Cl2-phenyl | Viscous liquid |
| 40 | CH3 | CH3 | propargyl | OCH2 | 2,5-Cl2-phenyl | Viscous liquid |
| 41 | CH3 | CH3 | CH2OCH3 | OCH2 | 2-Me-phenyl | Viscous liquid |
| 42 | CH3 | CH3 | propargyl | OCH2 | 2-Me-phenyl | Viscous liquid |
| 43 | CH3 | CH3 | CH2OCH3 | OCH2 | 3-Me-phenyl | Viscous liquid |
| 44 | CH3 | CH3 | propargyl | OCH2 | 3-Me-phenyl | Viscous liquid |
| 45 | CH3 | CH3 | propargyl | OCH2 | 4-Me-phenyl | Viscous liquid |
| 46 | CH3 | CH3 | propargyl | OCH2 | 2,4-Me2-phenyl | Viscous liquid |
| 47 | CH3 | CH3 | CH2OCH3 | OCH2 | 2,4-Me2-phenyl | Viscous liquid |
| 48 | CH3 | CH3 | H | OCH2 | 2,5-Me2-phenyl | White crystal |
| 49 | CH3 | CH3 | CH2OCH3 | OCH2 | 2,5-Me2-phenyl | Viscous liquid |
| 50 | CH3 | CH3 | propargyl | OCH2 | 2,5-Me2-phenyl | Viscous liquid |
| 51 | CH3 | CH3 | CH2OCH3 | OCH2 | 2-Me-5-Cl-phenyl | Viscous liquid |
| 52 | CH3 | CH3 | propargyl | OCH2 | 2-Me-5-Cl-phenyl | Viscous liquid |
| 53 | CH3 | CH3 | CH2OCH3 | OCH2 | 2-Me-5-SMe-phenyl | Viscous liquid |
| 54 | CH3 | CH3 | propargyl | OCH2 | 2-CHF2O-phenyl | Viscous liquid |
| 55 | CH3 | CH3 | CH2OCH3 | OCH2 | 2-CHF2O-phenyl | Viscous liquid |

Table 1 (Continued)

| | | | | | | |
|---|---|---|---|---|---|---|
| 56 | CH3 | CH3 | propargyl | OCH2 | 3-CHF2O-phenyl | Viscous liquid |
| 57 | CH3 | CH3 | CH2OCH3 | OCH2 | 3-CHF2O-phenyl | Viscous liquid |
| 58 | CH3 | CH3 | propargyl | OCH2 | 4-CHF2O-phenyl | Viscous liquid |
| 59 | CH3 | CH3 | CH2OCH3 | OCH2 | 4-CHF2O-phenyl | Viscous liquid |
| 60 | CH3 | CH3 | propargyl | OCH2 | 2-Me-4-CHF2O-phenyl | Viscous liquid |
| 61 | CH3. | CH3 | CH2OCH3 | OCH2 | 2-Me-5-CHF2O-phenyl | Viscous liquid |
| 62 | CH3 | CH3 | H | OCH2 | 2-Cl-4-CHF2O-phenyl | White crystal |
| 63 | CH3 | CH3 | CH2OCH3 | OCH2 | 2-Cl-4-CHF2O-phenyl | Viscous liquid |
| 64 | CH3 | CH3 | propargyl | OCH2 | 2-Cl-4-CHF2O-phenyl | Viscous liquid |
| 65 | CH3 | CH3 | H | OCH2 | 2-Cl-5-CHF2O-phenyl | Viscous liquid |
| 66 | CH3 | CH3 | CH2OCH3 | OCH2 | 2-Cl-5-CHF2O-phenyl | Viscous liquid |
| 67 | CH3 | CH3 | propargyl | OCH2 | 2-Cl-5-CHF2O-phenyl | Viscous liquid |
| 68 | CH3 | CH3 | H | OCH2 | 5-Cl-2-CHF2O-phenyl | Viscous |
| 69 | CH3 | CH3 | CH2OCH3 | OCH2 | 5-Cl-2-CHF2O-phenyl | Viscous liquid |
| 70 | CH3 | CH3 | propargyl | OCH2 | 5-Cl-2-CHF2O-phenyl | Viscous liquid |
| 71 | CH3 | CH3 | H | OCH2 | 2,5-(CHF2O)2-phenyl | White crystal |
| 72 | CH3 | CH3 | CH2OCH3 | OCH2 | 2,5-(CHF2O)2-phenyl | Viscous liquid |
| 73 | CH3 | CH3 | propargyl | OCH2 | 2,5-(CHF2O)2-phenyl | Viscous liquid |
| 74 | CH3 | CH3 | propargyl | OCH2 | 2,4-(CHF2O)2-phenyl | Viscous liquid |
| 75 | CH3 | CH3 | C2H5 | OCH2 | 3-PhO-phenyl | Viscous liquid |
| 76 | CH3 | CH3 | CH2OCH3 | OCH2 | 3-PhO-phenyl | Viscous liquid |
| 77 | CH3 | CH3 | propargyl | OCH2 | 3-PhO-phenyl | Viscous liquid |
| 78 | CH3 | CH3 | propargyl | OCH2 | 2-CF3-phenyl | Viscous liquid |
| 79 | CH3 | CH3 | propargyl | OCH2 | 3-CF3-phenyl | Viscous liquid |
| 80 | CH3 | CH3 | propargyl | OCH2 | 4-CF3-phenyl | Viscous liquid |
| 81 | CH3 | CH3 | CH2OCH3 | OCH2 | 2,5-(CF3)2-phenyl | Viscous liquid |
| 82 | CH3 | CH3 | propargyl | OCH2 | 2,5-(CF3)2-phenyl | Viscous liquid |
| 83 | CH3 | CH3 | CH2OCH3 | OCH2 | 3,5-(CF3)2-phenyl | Viscous liquid |
| 84 | CH3 | CH3 | propargyl | OCH2 | 3,5-(CF3)2-phenyl | Viscous liquid |
| 85 | CH3 | CH3 | propargyl | OCH2 | 3-benzyloxy-phenyl | Viscous liquid |

Table 1 (Continued)

| | | | | | | |
|---|---|---|---|---|---|---|
| 86 | CH3 | CH3 | propargyl | OCH2 | 2-naphthyl | Viscous liquid |
| 87 | CH3 | CH3 | CH2OCH3 | OCH2 | 2-naphthyl | Viscous liquid |
| 88 | CH3 | CH3 | propargyl | OCH2 | 3-naphthyl | Viscous liquid |
| 89 | CH3 | CH3 | CH2OCH3 | OCH2 | 3-naphthyl | Viscous liquid |
| 90 | CH3 | CH3 | propargyl | OCH2 | 2,2-Me2-dihydrobenzofuran-5-yl | Viscous liquid |
| 91 | CH3 | CH3 | H | CH2O | Phenyl | White crystal |
| 92 | CH3 | CH3 | CH2OCH3 | CH2O | Phenyl | Viscous liquid |
| 93 | CH3 | CH3 | propargyl | CH2O | Phenyl | Viscous liquid |
| 94 | CH3 | CH3 | CH2SCH3 | CH2O | Phenyl | Viscous liquid |
| 95 | CH3 | CH3 | H | CH2O | 3-Cl-phenyl | White crystal |
| 96 | CH3 | CH3 | C2H5 | CH2O | 3-Cl-phenyl | Viscous liquid |
| 97 | CH3 | CH3 | CH2OCH3 | CH2O | 3-Cl-phenyl | Viscous liquid |
| 98 | CH3 | CH3 | CH2SCH3 | CH2O | 3-Cl-phenyl | Viscous liquid |
| 99 | CH3 | CH3 | propargyl | CH2O | 3-Cl-phenyl | Viscous liquid |
| 100 | CH3 | CH3 | propargyl | CH2O | 2-Cl-phenyl | Viscous liquid |
| 101 | CH3 | CH3 | CH2OCH3 | CH2O | 2-Cl-phenyl | Viscous liquid |
| 102 | CH3 | CH3 | propargyl | CH2O | 4-Cl-phenyl | Viscous liquid |
| 103 | CH3 | CH3 | CH2OCH3 | CH2O | 4-Cl-phenyl | Viscous liquid |
| 104 | CH3 | CH3 | propargyl | CH2O | 2,4-Cl2-phenyl | Viscous liquid |
| 105 | CH3 | CH3 | CH2OCH3 | CH2O | 2,4-Cl2-phenyl | Viscous liquid |
| 106 | CH3 | CH3 | H | CH2O | 2,5-Cl2-phenyl | White crystal |
| 107 | CH3 | CH3 | C2H5 | CH2O | 2,5-Cl2-phenyl | Viscous liquid |
| 108 | CH3 | CH3 | CH2OCH3 | CH2O | 2,5-Cl2-phenyl | Viscous liquid |
| 109 | CH3 | CH3 | CH2SCH3 | CH2O | 2,5-Cl2-phenyl | Viscous liquid |
| 110 | CH3 | CH3 | propargyl | CH2O | 2,5-Cl2-phenyl | Viscous liquid |
| 111 | CH3 | CH3 | CH2OCH3 | CH2O | 2-Me-phenyl | Viscous liquid |
| 112 | CH3 | CH3 | propargyl | CH2O | 2-Me-phenyl | Viscous liquid |
| 113 | CH3 | CH3 | CH2OCH3 | CH2O | 3-Me-phenyl | Viscous liquid |
| 114 | CH3 | CH3 | propargyl | CH2O | 3-Me-phenyl | Viscous liquid |
| 115 | CH3 | CH3 | propargyl | CH2O | 4-Me-phenyl | Viscous liquid |

Table 1 (Continued)

| | | | | | | |
|---|---|---|---|---|---|---|
| 116 | CH3 | CH3 | H | CH2O | 2,4-Me2-phenyl | Viscous liquid |
| 117 | CH3 | CH3 | propargyl | CH2O | 2,4-Me2-phenyl | Viscous liquid |
| 118 | CH3 | CH3 | H | CH2O | 2,5-Me2-phenyl | White crystal |
| 119 | CH3 | CH3 | CH2OCH3 | CH2O | 2,5-Me2-phenyl | Viscous liquid |
| 120 | CH3 | CH3 | propargyl | CH2O | 2,5-Me2-phenyl | Viscous liquid |
| 121 | CH3 | CH3 | CH2OCH3 | CH2O | 2-Me-5-Cl-phenyl | Viscous liquid |
| 122 | CH3 | CH3 | propargyl | CH2O | 2-Me-5-Cl-phenyl | Viscous liquid |
| 123 | CH3 | CH3 | CH2OCH3 | CH2O | 2-Me-5-SMe-phenyl | Viscous liquid |
| 124 | CH3 | CH3 | propargyl | CH2O | 2-CHF2O-phenyl | Viscous liquid |
| 125 | CH3 | CH3 | CH2OCH3 | CH2O | 2-CHF2O-phenyl | Viscous liquid |
| 126 | CH3 | CH3 | propargyl | CH2O | 3-CHF2O-phenyl | Viscous liquid |
| 127 | CH3 | CH3 | CH2OCH3 | CH2O | 3-CHF2O-phenyl | Viscous liquid |
| 128 | CH3 | CH3 | propargyl | CH2O | 4-CHF2O-phenyl | Viscous liquid |
| 129 | CH3 | CH3 | CH2OCH3 | CH2O | 4-CHF2O-phenyl | Viscous liquid |
| 130 | CH3 | CH3 | propargyl | CH2O | 2-Me-4-CHF2O-phenyl | Viscous liquid |
| 131 | CH3 | CH3 | CH2OCH3 | CH2O | 2-Me-5-CHF2O-phenyl | Viscous liquid |
| 132 | CH3 | CH3 | H | CH2O | 2-Cl-4-CHF2O-phenyl | White crystal |
| 133 | CH3 | CH3 | CH2OCH3 | CH2O | 2-Cl-4-CHF2O-phenyl | Viscous liquid |
| 134 | CH3 | CH3 | propargyl | CH2O | 2-Cl-4-CHF2O-phenyl | Viscous liquid |
| 135 | CH3 | CH3 | H | CH2O | 2-Cl-5-CHF2O-phenyl | White crystal |
| 136 | CH3 | CH3 | CH2OCH3 | CH2O | 2-Cl-5-CHF2O-phenyl | Viscous liquid |
| 137 | CH3 | CH3 | propargyl | CH2O | 2-Cl-5-CHF2O-phenyl | Viscous liquid |
| 138 | CH3 | CH3 | H | CH2O | 5-Cl-2-CHF2O-phenyl | White crystal |
| 139 | CH3 | CH3 | CH2OCH3 | CH2O | 5-Cl-2-CHF2O-phenyl | Viscous liquid |
| 140 | CH3 | CH3 | propargyl | CH2O | 5-Cl-2-CHF2O-phenyl | Viscous liquid |
| 141 | CH3 | CH3 | H | CH2O | 2,5-(CHF2O)2-phenyl | White crystal |
| 142 | CH3 | CH3 | CH2OCH3 | CH2O | 2,5-(CHF2O)2-phenyl | Viscous liquid |
| 143 | CH3 | CH3 | propargyl | CH2O | 2,5-(CHF2O)2-phenyl | Viscous liquid |
| 144 | CH3 | CH3 | propargyl | CH2O | 2,4-(CHF2O)2-phenyl | Viscous liquid |
| 145 | CH3 | CH3 | H | CH2O | 3-PhO-phenyl | White crystal |

Table 1 (Continued)

| | | | | | | |
|---|---|---|---|---|---|---|
| 146 | CH3 | CH3 | CH2OCH3 | CH2O | 3-PhO-phenyl | Viscous liquid |
| 147 | CH3 | CH3 | propargyl | CH2O | 3-PhO-phenyl | Viscous liquid |
| 148 | CH3 | CH3 | propargyl | CH2O | 2-CF3-phenyl | Viscous liquid |
| 149 | CH3 | CH3 | CH2OCH3 | CH2O | 3-CF3-phenyl | Viscous liquid |
| 150 | CH3 | CH3 | propargyl | CH2O | 4-CF3-phenyl | Viscous liquid |
| 151 | CH3 | CH3 | CH2OCH3 | CH2O | 2,5-(CF3)2-phenyl | Viscous liquid |
| 152 | CH3 | CH3 | propargyl | CH2O | 2,5-(CF3)2-phenyl | Viscous liquid |
| 153 | CH3 | CH3 | CH2OCH3 | CH2O | 3,5-(CF3)2-phenyl | Viscous liquid |
| 154 | CH3 | CH3 | propargyl | CH2O | 3,5-(CF3)2-phenyl | Viscous liquid |
| 155 | CH3 | CH3 | propargyl | CH2O | 3-benzyloxy-phenyl | Viscous liquid |
| 156 | CH3 | CH3 | propargyl | CH2O | 2-naphthyl | Viscous liquid |
| 157 | CH3 | CH3 | CH2OCH3 | CH2O | 2-naphthyl | Viscous liquid |
| 158 | CH3 | CH3 | propargyl | CH2O | 3-naphthyl | Viscous liquid |
| 159 | CH3 | CH3 | CH2OCH3 | CH2O | 3-naphthyl | Viscous liquid |
| 160 | CH3 | CH3 | propargyl | CH2O | 2,2-Me2-dihydrobenzofuran-5-yl | Viscous liquid |
| 161 | CH3 | CH3 | H | CH2ON=C(CH3) | phenyl | White crystal |
| 162 | CH3 | CH3 | CH2OCH3 | CH2ON=C(CH3) | phenyl | Viscous liquid |
| 163 | CH3 | CH3 | propargyl | CH2ON=C(CH3) | phenyl | Viscous liquid |
| 164 | CH3 | CH3 | H | CH2ON=C(CH3) | 3-CF3-phenyl | White crystal |
| 165 | CH3 | CH3 | CH2OCH3 | CH2ON=C(CH3) | 3-CF3-phenyl | Viscous liquid |
| 166 | CH3 | CH3 | propargyl | CH2ON=C(CH3) | 3-CF3-phenyl | Viscous liquid |
| 167 | CH3 | CH3 | H | CH2ON=C(CH3) | 4-CF3-phenyl | White crystal |
| 168 | CH3 | CH3 | CH2OCH3 | CH2ON=C(CH3) | 4-CF3-phenyl | Viscous liquid |
| 169 | CH3 | CH3 | propargyl | CH2ON=C(CH3) | 4-CF3-phenyl | Viscous liquid |
| 170 | CH3 | CH3 | H | CH2ON=C(CH3) | 3-Cl-phenyl | White crystal |
| 171 | CH3 | CH3 | propargyl | CH2ON=C(CH3) | 3-Cl-phenyl | Viscous liquid |
| 172 | CH3 | CH3 | H | CH2ON=C(CH3) | 4-Cl-phenyl | White crystal |
| 173 | CH3 | CH3 | propargyl | CH2ON=C(CH3) | 4-Cl-phenyl | Viscous liquid |
| 174 | CH3 | CH3 | H | CH2ON=C(CH3) | 3-CHF2O-phenyl | White crystal |
| 175 | CH3 | CH3 | propargyl | CH2ON=C(CH3) | 3-CHF2O-phenyl | Viscous liquid |

Table 1 (Continued)

| | | | | | | |
|---|---|---|---|---|---|---|
| 176 | CH3 | CH3 | H | CH2ON=C(CH3) | 4-CHF2O-phenyl | White crystal |
| 177 | CH3 | CH3 | propargyl | CH2ON=C(CH3) | 4-CHF2O-phenyl | Viscous liquid |
| 178 | CH3 | CH3 | H | CH2ON=C(CH3) | 3-MeO-4-CHF2O-phenyl | White crystal |
| 179 | CH3 | CH3 | propargyl | CH2ON=C(CH3) | 3-MeO-4-CHF2O-phenyl | Viscous liquid |
| 180 | CH3 | CH3 | H | CH2ON=C(CH3) | 3-Me-4-CHF2O-phenyl | White crystal |
| 181 | CH3 | CH3 | propargyl | CH2ON=C(CH3) | 3-Me-4-CHF2O-phenyl | Viscous liquid |
| 182 | CH3 | CH3 | H | CH2ON=C(CH3) | 3-Cl-4-CHF2O-phenyl | White crystal |
| 183 | CH3 | CH3 | propargyl | CH2ON=C(CH3) | 3-Cl-4-CHF2O-phenyl | Viscous liquid |
| 184 | CH3 | CH3 | propargyl | CH2ON=C(CH3) | 2-naphthyl | Viscous liquid |
| 185 | CH3 | CH3 | propargyl | CH2ON=C(CH3) | 3-CF3O-phenyl | Viscous liquid |
| 186 | CH3 | CH3 | propargyl | CH2ON=C(CH3) | 4-CF3O-phenyl | Viscous liquid |
| 187 | CH3 | CH3 | propargyl | CH2ON=C(CH3) | 3-MeS-phenyl | Viscous liquid |
| 188 | CH3 | C2H5 | propargyl | CH2ON=C(CH3) | 3-CF3-phenyl | Viscous liquid |
| 189 | CH3 | H | propargyl | CH2ON=C(CH3) | 3-CF3-phenyl | Viscous liquid |
| 190 | C2H5 | CH3 | propargyl | CH2ON=C(CH3) | 3-CF3-phenyl | Viscous liquid |
| 191 | CH3 | CH3 | H | CH=NOCH(CH3) | phenyl | White crystal |
| 192 | CH3 | CH3 | CH2OCH3 | CH=NOCH(CH3) | phenyl | Viscous liquid |
| 193 | CH3 | CH3 | propargyl | CH=NOCH(CH3) | phenyl | Viscous liquid |
| 194 | CH3 | CH3 | H | CH=NOCH(CH3) | 3-CF3-phenyl | White crystal |
| 195 | CH3 | CH3 | CH2OCH3 | CH=NOCH(CH3) | 3-CF3-phenyl | Viscous liquid |
| 196 | CH3 | CH3 | propargyl | CH=NOCH(CH3) | 3-CF3-phenyl | Viscous liquid |
| 197 | CH3 | CH3 | H | CH=NOCH(CH3) | 4-CF3-phenyl | White crystal |
| 198 | CH3 | CH3 | CH2OCH3 | CH=NOCH(CH3) | 4-CF3-phenyl | Viscous liquid |
| 199 | CH3 | CH3 | propargyl | CH=NOCH(CH3) | 4-CF3-phenyl | Viscous liquid |
| 200 | CH3 | CH3 | H | CH=NOCH(CH3) | 3-Cl-phenyl | White crystal |
| 201 | CH3 | CH3 | propargyl | CH=NOCH(CH3) | 3-Cl-phenyl | Viscous liquid |
| 202 | CH3 | CH3 | H | CH=NOCH(CH3) | 4-Cl-phenyl | White crystal |
| 203 | CH3 | CH3 | propargyl | CH=NOCH(CH3) | 4-Cl-phenyl | Viscous liquid |
| 204 | CH3 | CH3 | H | CH=NOCH(CH3) | 3-CHF2O-phenyl | White crystal |
| 205 | CH3 | CH3 | propargyl | CH=NOCH(CH3) | 3-CHF2O-phenyl | Viscous liquid |

Table 1 (Continued)

| | | | | | | |
|---|---|---|---|---|---|---|
| 206 | CH3 | CH3 | H | CH=NOCH(CH3) | 4-CHF2O-phenyl | White crystal |
| 207 | CH3 | CH3 | propargyl | CH=NOCH(CH3) | 4-CHF2O-phenyl | Viscous liquid |
| 208 | CH3 | CH3 | H | CH=NOCH(CH3) | 3-MeO-4-CHF2O-phenyl | White crystal |
| 209 | CH3 | CH3 | propargyl | CH=NOCH(CH3) | 3-MeO-4-CHF2O-phenyl | Viscous liquid |
| 210 | CH3 | CH3 | H | CH=NOCH(CH3) | 3-Me-4-CHF2O-phenyl | White crystal |
| 211 | CH3 | CH3 | propargyl | CH=NOCH(CH3) | 3-Me-4-CHF2O-phenyl | Viscous liquid |
| 212 | CH3 | CH3 | H | CH=NOCH(CH3) | 3-Cl-4-CHF2O-phenyl | White crystal |
| 213 | CH3 | CH3 | propargyl | CH=NOCH(CH3) | 3-Cl-4-CHF2O-phenyl | Viscous liquid |
| 214 | CH3 | CH3 | propargyl | CH=NOCH(CH3) | 2-naphthyl | Viscous liquid |
| 215 | CH3 | CH3 | propargyl | CH=NOCH(CH3) | 3-CF3O-phenyl | Viscous liquid |
| 216 | CH3 | CH3 | propargyl | CH=NOCH(CH3) | 4-CF3O-phenyl | Viscous liquid |
| 217 | CH3 | CH3 | propargyl | CH=NOCH(CH3) | 3-MeS-phenyl | Viscous liquid |
| 218 | CH3 | CH3 | propargyl | CH=NOCH2 | 3-CF3-phenyl | Viscous liquid |
| 219 | CH3 | H | propargyl | CH=NOCH(CH3) | 3-CF3-phenyl | Viscous liquid |
| 220 | CH3 | C2H5 | propargyl | CH=NOCH(CH3) | 3-CF3-phenyl | Viscous liquid |
| 221 | CH3 | CH3 | H | CH2S | phenyl | White crystal |
| 222 | CH3 | CH3 | propargyl | CH2S | phenyl | Viscous liquid |
| 223 | CH3 | CH3 | H | CH2S | 2,5-Cl2phenyl | White crystal |
| 224 | CH3 | CH3 | propargyl | CH2S | 2,5-Cl2phenyl | Viscous liquid |
| 225 | CH3 | CH3 | H | CH2S | 2-Cl-5-CHF2O-phenyl | White crystal |
| 226 | CH3 | CH3 | propargyl | CH2S | 2-Cl-5-CHF2O-phenyl | Viscous liquid |
| 227 | CH3 | CH3 | propargyl | CH2S | 4-CF3-pyridin-2-yl | Viscous liquid |
| 228 | CH3 | CH3 | H | CH2S | 6-Cl-4-CF3-pyridin-2-yl | Viscous liquid |
| 229 | CH3 | CH3 | propargyl | CH2S | 6-Cl-4-CF3-pyridin-2-yl | Viscous liquid |
| 230 | CH3 | CH3 | H | CH2S | benzothiazol-2-yl | White crystal |
| 231 | CH3 | CH3 | H | C≡C | 3-CF3-Phenyl | White crystal |
| 232 | CH3 | CH3 | propargyl | C≡C | 3-CF3-Phenyl | Viscous liquid |
| 233 | CH3 | CH3 | CH2OCH3 | C≡C | 3-CF3-Phenyl | Viscous liquid |
| 234 | CH3 | CH3 | propargyl | CH=CH | Phenyl | Viscous liquid |
| 235 | CH3 | CH3 | CH2OCH3 | CH=CH | Phenyl | Viscous liquid |

In Table 1, Me indicates a methyl group and Ph indicates a phenyl group.
The value obtained in the analysis of the resulting compound by means of $^1$H-NMR is as follows.

$^1$H-NMR data ($CDCl_3$) of the compound No. 21; 2.13 (3H, s), 3.59 (3H, s), 3.72 (3H, s), 4.95 (2H, s), 5.72 (1H, brs), 7.35 (5H, brs)
$^1$H-NMR data ($CDCl_3$) of the compound No. 22; 2.14 (3H, s), 3.6 (3H, s), 3.65 (3H, brs), 3.72 (3H, s), 4.4 (2H, brs), 4.95 (2H, s), 5.72 (1H, brs), 7.35 (5H, brs)
$^1$H-NMR data ($CDCl_3$) of the compound No. 23; 2.23 (3H, s), 2.24 (1H, dd), 3.62 (3H, s), 3.67 (3H, s), 3.93 (1H, dd), 4.63 (1H, dd), 4.85 (2H, s), 7.34 (5H, m)
$^1$H-NMR data ($CDCl_3$) of the compound No. 36; 2.19 (3H, s), 3.64 (3H, s), 3.76 (3H, s), 4.95 (2H, s), 6.07 (1H, brs), 7.22 (1H, dd), 7.33 (1H, d), 7.53 (1H, d)
$^1$H-NMR data ($CDCl_3$) of the compound No. 37; 1.85 (3H, t), 2.19 (3H, s), 3.64 (3H, s), 3.76 (3H, s), 4.7 (2H, br), 4.95 (2H, s), 7.22 (1H, dd), 7.33 (1H, d), 7.53 (1H, d)
$^1$H-NMR data ($CDCl_3$) of the compound No. 38; 2.2 (3H, s), 3.55 (3H, s), 3.6 (3H, brs), 3.70 (3H, s), 4.12 (1H, dd), 4.65 (1H, dd), 4.92 (2H, s), 7.23 (1H, d), 7.31 (1H, d), 7.50 (1H, s)
$^1$H-NMR data ($CDCl_3$) of the compound No. 40; 2.21 (3H, s), 2.27 (1H, dd), 3.64 (3H, s), 3.70 (3H, s), 4.12 (1H, dd), 4.65 (1H, dd), 4.92 (2H, s), 7.23 (1H, d), 7.31 (1H, d), 7.50 (1H, s)
$^1$H-NMR data ($CDCl_3$) of the compound No. 48; 2.18 (3H, s), 2.30 (3H, s), 2.34 (3H, s), 3.59 (3H, s), 3.72 (3H, s), 4.83 (2H, s), 6.55 (1H, brs), 7.0-7.2 (3H, m)
$^1$H-NMR data ($CDCl_3$) of the compound No. 50; 2.17 (3H, s), 2.24 (1H, dd), 2.31 (3H, s), 2.33 (3H, s), 3.64 (3H, s), 3.70 (3H, s), 3.89 (1H, d), 4.88 (1H, d), 4.79 (1H, d), 4.86 (1H, d), 7.08 (3H, m)
$^1$H-NMR data ($CDCl_3$) of the compound No. 65; 2.18 (3H, s), 3.63 (3H, s), 3.73 (3H, s), 4.97 (2H, s), 6.09 (1H, brs), 6.52 (1H, t), 7.05 (1H, dd), 7.30 (1H, d), 7.38 (1H, d)
$^1$H-NMR data ($CDCl_3$) of the compound No. 66; 2.19 (3H, s), 3.39 (3H, s), 3.61 (3H, s), 3.71 (3H, brs), 4.74 (1H, d), 4.93 (2H, s), 5.03 (1H, d), 6.52 (1H, t), 7.04 (1H, dd), 7.31 (1H, d), 7.36 (1H, d)
$^1$H-NMR data ($CDCl_3$) of the compound No. 67; 2.20 (3H, s), 2.26 (1H, t), 3.64 (3H, s), 3.69 (3H, brs), 4.08 (1H, dd), 4.62 (1H, dd), 4.88 (2H, s), 6.48 (1H, t), 7.09 (1H, dd), 7.32 (1H, d), 7.50 (1H, d)
$^1$H-NMR data ($CDCl_3$) of the compound No. 68; 2.17 (3H, s), 3.62 (3H, s), 3.74 (3H, s), 4.90 (2H, s), 6.09 (1H, brs), 6.46 (1H, t), 7.09 (1H, d), 7.31 (1H, dd), 7.50 (1H, d)
$^1$H-NMR data ($CDCl_3$) of the compound No. 69; 2.18 (3H, s), 3.39 (3H, s), 3.61 (3H, s), 3.73 (3H, brs), 4.70 (1H, d), 4.87 (2H, s), 5.03 (1H, d), 6.47 (1H, t), 7.09 (1H, d), 7.31 (1H, dd), 7.50 (1H, d)
$^1$H-NMR data ($CDCl_3$) of the compound No. 70; 2.19 (3H, s), 2.26 (1H, t), 3.63 (3H, s), 3.70 (3H, brs), 4.08 (1H, dd), 4.62 (1H, dd), 4.88 (2H, s), 6.48 (1H, t), 7.09 (1H, d), 7.32 (1H, dd), 7.50 (1H, d)
$^1$H-NMR data ($CDCl_3$) of the compound No. 71; 2.17 (3H, s), 3.62 (3H, s), 3.74 (3H, s), 4.90 (2H, s), 6.09 (1H, brs), 6.46 (1H, t), 6.53 (1H, t), 7.09 (1H, d), 7.31 (1H, dd), 7.50 (1H, d)
$^1$H-NMR data ($CDCl_3$) of the compound No. 72; 2.18 (3H, s), 3.39 (3H, s), 3.61 (3H, s), 3.73 (3H, brs), 4.70 (1H, d), 4.87 (2H, s), 5.03 (1H, d), 6.47 (1H, t), 6.56 (1H, t), 7.09 (1H, d), 7.31 (1H, dd), 7.50 (1H, d)
$^1$H-NMR data ($CDCl_3$) of the compound No. 73; 2.19 (3H, s), 2.26 (1H, t), 3.63 (3H, s), 3.70 (3H, brs), 4.08 (1H, dd), 4.62 (1H, dd), 4.88 (2H, s), 6.48 (1H, t), 6.56 (1H, t), 7.09 (1H, d), 7.32 (1H, dd), 7.50 (1H, d)
$^1$H-NMR data ($CDCl_3$) of the compound No. 75; 1.13 (3H, t), 2.08 (3H, s), 3.54 (3H, s), 3.60 (2H, br), 3.61 (3H, brs), 4.75 (1H, d), 4.82 (1H, d), 6.9-7.2 (6H, m), 7.3-7.4 (3H, m)
$^1$H-NMR data ($CDCl_3$) of the compound No. 76; 2.10 (3H, s), 3.37 (3H, s), 3.67 (3H, brs), 4.63 (1H, d), 4.80 (2H, s), 5.04 (1H, d), 6.95-7.16 (6H, m), 7.3-7.4 (3H, m)
$^1$H-NMR data ($CDCl_3$) of the compound No. 77; 2.11 (3H, s), 2.22 (1H, t), 3.62 (3H, s), 3.65 (3H, brs), 3.96 (1H, dd), 4.63 (1H, dd), 4.81 (2H, s), 6.92-7.18 (6H, m), 7.3-7.4 (3H, m)
$^1$H-NMR data ($CDCl_3$) of the compound No. 116; 2.14 (3H, s), 2.25 (3H, s), 2.26 (3H, s), 3.69 (3H, s), 3.75 (3H, s), 4.78 (2H, s), 6.25 (1H, brs), 6.80 (1H, d), 6.96 (2H, m)
$^1$H-NMR data ($CDCl_3$) of the compound No. 117; 2.13 (3H, s), 2.23 (1H, t), 2.26 (3H, s), 2.27 (3H, s), 3.68 (3H, brs), 3.71 (3H, s), 4.16 (1H, brd), 4.62 (1H, brd), 4.74 (2H, d), 6.78 (1H, d), 6.95 (1H, d), 6.96 (1H, s)
$^1$H-NMR data ($CDCl_3$) of the compound No. 164; 2.28 (3H, s), 2.33 (3H, s), 3.6 (3H, s), 3.7 (3H, s), 4.78 (2H, s), 6.2 (1H, brs), 7.2-7.8 (4H, m)
$^1$H-NMR data ($CDCl_3$) of the compound No. 165; 2.20 (3H, s), 2.32 (3H, s), 3.70 (6H, s), 3.75 (3H, brs), 4.22 (1H, dd), 4.75 (1H, dd), 4.94 (1H, d), 5.02 (1H, d), 7.48 (1H, t), 7.60 (1H, d), 7.79 (1H, d), 7.88 (1H, s)
$^1$H-NMR data ($CDCl_3$) of the compound No. 166; 2.20 (3H, s), 2.26 (1H, d), 2.32 (3H, s), 3.70 (6H, s), 4.22 (1H, dd), 4.75 (1H, dd), 4.94 (1H, d), 5.02 (1H, d), 7.48 (1H, t), 7.60 (1H, d), 7.79 (1H, d), 7.88 (1H, s)
$^1$H-NMR data ($CDCl_3$) of the compound No. 167; 2.21 (3H, s), 2.28 (3H, s), 3.69 (3H, s), 3.79 (3H, s), 4.95 (2H, s), 7.64 (2H, d), 7.71 (2H, d)
$^1$H-NMR data ($CDCl_3$) of the compound No. 169; 2.20 (3H, d), 2.27 (1H, t), 2.32 (3H, s), 3.70 (6H, brs), 4.23 (1H,

dd), 4.77 (1H, dd), 4.94 (1H, d), 5.03 (1H, d), 7.61 (2H, d), 7.73 (2H, d)

$^1$H-NMR data ($CDCl_3$) of the compound No. 194; 1.58 (3H, d), 2.21 (3H, s), 3.69 (3H, s), 5.23 (1H, q), 7.08 (1H, brs), 7.4-7.6 (4H, m), 8.01 (1H, s)

$^1$H-NMR data ($CDCl_3$) of the compound No. 196; 1.58 (3H, d), 2.18 (1H, dd), 2.26 (3H, s), 3.68 (3H, s), 3.90 (1H, dd), 4.5 (1H, dd), 5.22 (1H, q), 7.4-7.6 (4H, m), 7.97 (1H, d)

$^1$H-NMR data ($CDCl_3$) of the compound No. 197; 1.57 (3H, d), 2.21 (3H, s), 3.68 (3H, s), 3.69 (3H, s), 5.25 (1H, q), 7.08 (1H, brs), 7.46 (2H, d), 7.61 (2H, d), 8.01 (1H, s)

$^1$H-NMR data ($CDCl_3$) of the compound No. 199; 1.56 (3H, d), 2.20 (1H, dd), 2.25 (3H, s), 3.68 (6H, brs), 4.52 (1H, dd), 5.22 (1H, q), 7.45 (2H, m), 7.6 (2H, m), 7.96 (1H, s)

$^1$H-NMR data ($CDCl_3$) of the compound No. 228; 2.27 (3H, s), 3.68 (3H, s), 3.83 (3H, s), 4.14 (2H, s), 5.5 (1H, brs), 7.77 (1H, d), 8.63 (1H, d)

$^1$H-NMR data ($CDCl_3$) of the compound No. 229; 2.35 (3H, s), 2.48 (1H, d), 3.94 (1H, d), 4.10 (3H, s), 4.12 (3H, s), 4.58 (1H, d), 4.94 (2H, s), 7.75 (1H, s), 8.62 (1H, s)

$^1$H-NMR data ($CDCl_3$) of the compound No. 230; 2.25 (3H, s), 3.67 (3H, s), 3.91 (3H, s), 4.24 (2H, s), 7.32 (1H, t), 7.47 (1H, t), 7.74 (1H, d), 8.02 (1H, d), 9.6 (1H, brs)

$^1$H-NMR data ($CDCl_3$) of the compound No. 231; 2.33 (3H, s), 3.73 (3H, s), 3.83 (3H, s), 6.35 (1H, brs), 7.46 (1H, t), 7.56 (1H, d), 7.63 (1H, d), 7.72 (1H, s)

$^1$H-NMR data ($CDCl_3$) of the compound No. 232; 2.27 (1H, t), 2.35 (3H, s), 3.71 (3H, s), 3.79 (3H, brs), 4.25 (1H, d), 4.85 (1H, d), 7.46 (1H, t), 7.56 (1H, d), 7.61 (1H, d), 7.70 (1H, s)

$^1$H-NMR data ($CDCl_3$) of the compound No. 233; 2.34 (3H, s), 3.46 (3H, s), 3.69 (3H, s), 3.80 (3H, brs), 4.89 (1H, d), 5.25 (1H, d), 7.45 (1H, t), 7.54 (1H, d), 7.60 (1H, d), 7.69 (1H, s)

Formulation Example 1

20 Parts of the compound No. 2 described in Table 1, 75 parts of diatomaceous earth and 5 parts of a surfactant containing alkylbenzenesulfonic acid as a main ingredient were uniformly pulverized and mixed to give a wettable powder.

Formulation Example 2

30 Parts of the compound No. 3 described in Table 1, 15 parts of "Sorpol" 3005X (trade mark of Toho Kagaku Co., Ltd., mixture of nonionic surfactant and anionic surfactant), 25 parts of xylene and 30 parts of dimethylformamide were sufficiently mixed to give an emulsifiable concentrate.

The following Test Examples illustrate that the compound of the present invention is useful as an agricultural/horticultural fungicide

Test Example 1 Preventive activity on wheat powdery mildow

Wettable powders prepared according to the same manner as that described in Formulation Example 1 were diluted with water to the predetermined concentration and then applied by foliar application on wheat plants (var. Norin No. 61) at the 1 to 2 leaf stage grown in pots of 6 cm in diameter at a ratio of 10 ml per one pot. After the chemical solution was air-dried, a spore suspension obtained from the leaf of wheat infected with Erysiphe graminis was spray-inoculated. After inoculation, the seedling of wheat was allowed to stand at room temperature for 7 to 10 days.

For evaluation, the diseased area ratio of each leaf was measured and the preventive value was calculated from the following equation:

$$\text{The preventive value (\%)} = \frac{\text{(average diseased area ratio in untreated plot)} - \text{(average diseased area ratio in treated plot)}}{\text{(average diseased area ratio in untreated plot)}} \times 100$$

The results are shown in the column under the item "preventive value 1" in Table 2.

The test compound No. cited in the Table 2 corresponds to the compound No. of Table 1.

Test Example 2 Preventive activity on wheat brown

Wettable powders prepared according to the same manner as that described in Formulation Example 1 were diluted with water to the predetermined concentration and then applied by foliar application on wheat plants (var. Norin No. 61) at the 1 to 2 leaf stage grown in pots of 6 cm in diameter at a ratio of 10 ml per one pot. After the chemical solution was air-dried, a spore suspension obtained by pulverizing the leaf of wheat infected with Puccinia recondita was spray-inoculated. After inoculation, the wheat seedling was maintained in a moist chamber at 22°C for 15 hours and was allowed to stand on a water tank in a green house for 7 days.

For evaluation, the diseased area ratio of each leaf was measured and the preventive value was calculated from the following equation:

$$\text{The preventive value (\%)} = \frac{\text{(average diseased area ratio in untreated plot)} - \text{(average diseased area ratio in treated plot)}}{\text{(average diseased area ratio in untreated plot)}} \times 100$$

The results are shown in the column under the item "preventive value 2" in Table 2.

Table 2

| Test compound No. | Concentration of active ingredient (ppm) | Preventive value 1 (%) | Preventive value 2 (%) |
|---|---|---|---|
| 22 | 200 | 91 | 87 |
| 23 | 200 | 100 | 100 |
| 37 | 200 | 98 | 92 |
| 38 | 200 | 100 | 100 |
| 40 | 200 | 100 | 100 |
| 48 | 200 | 93 | 86 |
| 50 | 200 | 100 | 100 |
| 66 | 200 | 100 | 100 |
| 67 | 200 | 100 | 100 |
| 69 | 200 | 100 | 100 |
| 70 | 200 | 100 | 100 |
| 72 | 200 | 100 | 100 |
| 73 | 200 | 100 | 100 |
| 76 | 200 | 99 | 98 |
| 77 | 200 | 100 | 100 |
| 117 | 200 | 95 | 96 |
| 165 | 200 | 100 | 100 |
| 166 | 200 | 100 | 100 |
| 169 | 200 | 100 | 100 |
| 196 | 200 | 100 | 100 |
| 199 | 200 | 100 | 100 |
| 230 | 200 | 89 | 85 |
| 232 | 200 | 100 | 100 |

## Claims

**1.** A N-pyrazolyl carbamate derivative represented by the general formula (I):

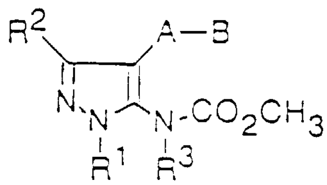

(I)

wherein $R^1$ and $R^2$ independently indicate a hydrogen atom or a $C_1$-$C_4$ alkyl group; $R^3$ is a hydrogen atom, a $C_1$-$C_4$ alkyl group, a $C_2$-$C_5$ alkynyl group, a $C_2$-$C_4$ alkylthioalkyl group or a $C_2$-$C_4$ alkoxyalkyl group; A is -O-, -C(O)-, $-OCH_2-$, $-CH_2O-$, $-CH_2S-$, -C≡C-, -CH=CH-, $-CH_2CH_2-$, $-CH_2ON=C(R^4)-$ or $-CH=NOC(R^4)(R^5)-$ (wherein $R^4$ and $R^5$ independently indicate a hydrogen atom or a $C_1$-$C_4$ alkyl group)); and B is a hydrogen atom, an aryl group selected from phenyl and naphthyl groups or a heterocyclic group selected from pyridyl, pyrimidyl, furanyl and benzothiazolyl groups, said aryl and heterocyclic groups being optionally substituted with a substituent selected from the group consisting of a cyano group; a nitro group ; a halogen atom; a $C_1$-$C_4$ alkyl group; a $C_1$-$C_4$ haloalkyl group; a $C_1$-$C_6$ alkoxy group optionally substituted with a halogen atom or a $C_3$-$C_6$ cycloalkyl group; a $C_1$-$C_6$ alkylthio group; a $C_2$-$C_6$ alkenyloxy group optionally substituted with a halogen atom; a $C_2$-$C_6$ alkynyloxy group; and a phenoxy, benzyloxy or pyridyloxy group optionally substituted with a cyano group, nitro group, a halogen atom, a $C_1$-$C_4$ alkyl group, a $C_1$-$C_4$ haloalkyl group, a $C_1$-$C_6$ alkoxy group optionally substituted with a halogen atom or a $C_3$-$C_6$ cycloalkyl group, $C_1$-$C_6$ alkylthio group, $C_2$-$C_6$ alkenyloxy group which may be substituted with a halogen atom, or a $C_2$-$C_6$ alkynyloxy group.

**2.** The N-pyrazolyl carbamate derivative according to claim 1, wherein $R^1$ and $R^2$ indicate a $C_1$-$C_4$ alkyl group; $R^3$ is a hydrogen atom, a $C_1$-$C_4$ alkyl group, a $C_2$-$C_5$ alkynyl group or a $C_2$-$C_4$ alkoxyalkyl group; and A is -O-,$-OCH_2-$, $-CH_2O-$, $-CH_2S-$, -C≡C-, -CH=CH-, $-CH_2ON=C(R^4)-$ or $-CH=NOC(R^4)(R^5)-$ (wherein $R^4$ and $R^5$ independently indicate a hydrogen atom or a $C_1$-$C_4$ alkyl group).

**3.** The N-pyrazolyl carbamate derivative according to claim 1, wherein $R^1$ and $R^2$ indicate a $C_1$-$C_4$ alkyl group; $R^3$ is a hydrogen atom, a $C_1$-$C_4$ alkyl group, a $C_2$-$C_5$ alkynyl group or a $C_2$-$C_4$ alkoxyalkyl group; A is -O-, $-OCH_2-$, $-CH_2O-$, $-CH_2S-$, -C≡C-, $-CH_2ON=C(R^4)-$ or $-CH=NOC(R^4)(R^5)-$(wherein $R^4$ and $R^5$ independently indicate a hydrogen atom or a $C_1$-$C_2$ alkyl group); and B is said aryl group or said heterocyclic group which may contain a substituent(s) selected from a halogen atom, a $C_1$-$C_4$ alkyl group, a $C_1$-$C_4$ haloalkyl group, a $C_1$-$C_6$ alkoxy group which may be substituted with a halogen atom and a phenoxy group.

**4.** The N-pyrazolyl carbamate derivative according to claim 1, wherein $R^1$ and $R^2$ indicate a methyl group; $R^3$ is a hydrogen atom, a $C_1$-$C_4$ alkyl group, a $C_2$-$C_5$ alkynyl group or a $C_2$-$C_4$ alkoxyalkyl group; A is $-OCH_2-$, $-CH_2O-$, $-CH_2S-$, -C≡C-, $-CH_2ON=C(R^4)-$, or $-CH=NOC(R^4)(R^5)-$ (wherein $R^4$ is a hydrogen atom or a methyl group and $R^5$ is a hydrogen atom); and B is a phenyl group which may contain a substituent(s) selected from the group consisting of a halogen atom, a $C_1$-$C_4$ alkyl group, a $C_1$-$C_4$ alkoxy group which may be substituted with a fluorine atom, a trifluoromethyl group and a phenoxy group.

**5.** A pyrazole-5-yl carboxylate derivative represented by the general formula:

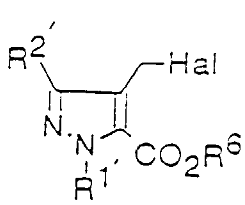

(VIII) or

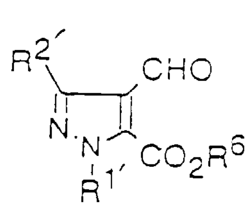

(XIII)

wheren $R^{1'}$ and $R^{2'}$ indicate a $C_1$-$C_4$ alkyl group; Hal is chlorine atom or a bromine atom; and $R^6$ is a $C_1$-$C_4$ alkyl group as an intermediate for the production of the N-pyrazolyl carbamate derivative of claim 2 or 3.

6. The 1,3-dimethylpyrazole-5-yl carboxylate derivative according to claim 5, wherein $R^{1'}$ and $R^{2'}$ indicate a methyl group.

7. An agricultural/horticultural fungicide comprising the N-pyrazolyl carbamate derivative according to any of claims 1-4 as an active ingredient.

**Patentansprüche**

1. N-Pyrazolylcarbamatderivat der allgemeinen Formel (I):

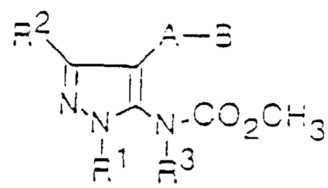

worin $R^1$ und $R^2$ unabhängig voneinander ein Wasserstoffatom oder eine $C_1$-$C_4$-Alkylgruppe bedeuten; $R^3$ ein Wasserstoffatom, eine $C_1$-$C_4$-Alkylgruppe, eine $C_2$-$C_5$-Alkinylgruppe, eine $C_2$-$C_4$-Alkylthioalkylgruppe oder eine $C_2$-$C_4$-Alkoxyalkylgruppe ist; A -O-, -C(O)-, $-CH_2-$, $-CH_2O-$, $-CH_2S-$,-C≡C-, -CH=CH-, $-CH_2CH_2-$, $-CH_2ON=C(R^4)-$ oder $-CH=NOC(R^4)(R^5)-$(worin $R^4$ und $R^5$ unabhängig voneinander ein Wasserstoffatom oder eine $C_1$-$C_4$-Alkylgruppe bedeuten) ist; und B ein Wasserstoffatom, eine aus Phenyl- und Naphthylgruppen gewählte Arylgruppe oder eine heterocyclische Gruppe ist, gewählt aus Pyridyl-, Pyrimidyl-, Furanyl und Benzothiazolylgruppen, wobei die Aryl- und heterocyclischen Gruppen wahlweise substituiert sind mit einem Substituenten, gewählt aus der Gruppe, bestehend aus einer Cyanogruppe; einer Nitrogruppe; einem Halogenatom; einer $C_1$-$C_4$-Alkylgruppe; einer $C_1$-$C_4$-Halogenalkylgruppe; einer $C_1$-$C_6$-Alkoxygruppe, wahlweise substituiert mit einem Halogenatom oder einer $C_3$-$C_6$-Cycloalkylgruppe; einer $C_1$-$C_6$-Alkylthiogruppe; einer $C_2$-$C_6$-Alkenyloxygruppe, wahlweise substituiert mit einem Halogenatom; einer $C_2$-$C_6$-Alkinyloxygruppe; und einer Phenoxygruppe, Benzyloxygruppe oder Pyridyloxygruppe, wahlweise substituiert mit einer Cyanogruppe, Nitrogruppe, einem Halogenatom, einer $C_1$-$C_4$-Alkylgruppe, einer $C_1$-$C_4$-Halogenalkylgruppe, einer $C_1$-$C_6$-Alkoxygruppe, wahlweise substituiert mit einem Halogenatom oder einer $C_3$-$C_6$-Cycloalkylgruppe, einer $C_1$-$C_6$-Alkylthiogruppe, $C_2$-$C_6$-Alkenyloxygruppe, welche mit einem Halogenatom substituiert sein kann oder einer $C_2$-$C_6$-Alkinyloxygruppe.

2. N-Pyrazolylcarbamatderivat nach Anspruch 1, wobei $R^1$ und $R^2$ eine $C_1$-$C_4$-Alkylgruppe bedeutend $R^3$ ein Wasserstoffatom, eine $C_1$-$C_4$-Alkylgruppe, $C_2$-$C_5$-Alkinylgruppe oder $C_2$-$C_4$-Alkoxyalkylgruppe ist; und A -O-, $-OCH_2-$, $-CH_2O-$, $-CH_2S-$, -C≡C-, -CH=CH-, $-CH_2ON=C(R^4)-$ oder $-CH=NOC(R^4)(R^5)-$ (worin $R^4$ und $R^5$ unabhängig voneinander ein Wasserstoffatom oder eine $C_1$-$C_4$-Alkylgruppe bedeuten) ist.

3. N-Pyrazolylcarbamatderivat nach Anspruch 1, wobei $R^1$ und $R^2$ eine $C_1$-$C_4$-Alkylgruppe bedeuten; $R^3$ ein Wasserstoffatom, eine $C_1$-$C_4$-Alkylgruppe, $C_2$-$C_5$-Alkinylgruppe oder $C_2$-$C_4$-Alkoxyalkylgruppe ist; A -O-, $-OCH_2-$, $-CH_2O-$, $-CH_2S-$, -C≡C-, $-CH_2ON=C(R^4)-$ oder $-CH=NOC(R^4)(R^5)-$ (worin $R^4$ und $R^5$ unabhängig voneinander ein Wasserstoffatom oder eine $C_1$-$C_4$-Alkylgruppe bedeuten) ist; und B die Arylgruppe oder die heterocyclische Gruppe ist, welche (einen)Substituenten enthalten kann, gewählt aus einem Halogenatom, einer $C_1$-$C_4$-Alkylgruppe, $C_1$-$C_4$-Halogenalkylgruppe, $C_1$-$C_6$-Alkoxygruppe, welche mit einem Halogenatom substituiert sein kann, und einer Phenoxygruppe.

4. N-Pyrazolylcarbamatderivat nach Anspruch 1, wobei $R^1$ und $R^2$ eine Methylgruppe bedeuten; $R^3$ ein Wasserstoffatom, eine $C_1$-$C_4$-Alkylgruppe, $C_2$-$C_5$-Alkinylgruppe oder $C_2$-$C_4$-Alkoxyalkylgruppe ist; A $-OCH_2-$, $-CH_2O-$, $-CH_2S-$, -C≡C-, $CH_2ON=C(R^4)-$ oder $-CH=NOC(R^4)(R^5)-$ (worin $R^4$ ein Wasserstoffatom oder eine Methylgruppe ist und $R^5$ ein Wasserstoffatom ist) ist; und B eine Phenylgruppe ist, welche (einen) Substituenten enthalten kann, gewählt aus der ein Halogenatom, eine $C_1$-$C_4$-Alkylgruppe, $C_1$-$C_4$-Alkoxygruppe, die mit einem Fluoratom, einer Trifluormethylgruppe und einer Phenoxygruppe substituiert sein, kann, umfassenden Gruppe.

5. Pyrazol-5-yl-carboxylatderivat der allgemeinen Formel:

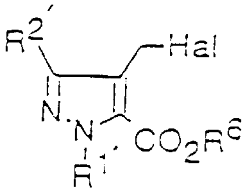 (VIII) oder 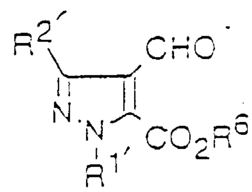 (XIII)

worin $R^{1'}$ und $R^{2'}$ eine $C_1$-$C_4$-Alkylgruppe bedeuten; Hal ein Chloratom oder ein Bromatom ist; und $R^6$ eine $C_1$-$C_4$-Alkylgruppe ist, als Zwischenverbindung zur Herstellung des N-Pyrazolylcarbamatderivats nach Anspruch 2 oder 3.

**6.** 1,3-Dimethylpyrazol-5-yl-carboxylatderivat nach Anspruch 5, wobei $R^{1'}$ und $R^{2'}$ eine Methylgruppe bedeuten.

**7.** Fungizid für Landwirtschaft/Gartenbau, umfassend das N-Pyrazolylcarbamatderivat nach mindestens einem der Ansprüche 1-4 als Wirkstoff.

## Revendications

**1.** Dérivé de N-pyrazolylcarbamate représenté par la formule générale (I) :

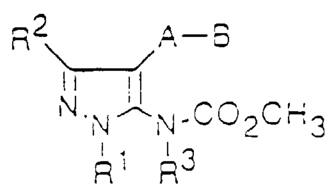

(I)

dans laquelle $R^1$ et $R^2$ représentent indépendamment un atome d'hydrogène ou un groupe alkyle en $C_1$-$C_4$; $R^3$ représente un atome d'hydrogène, un groupe alkyle en $C_1$-$C_4$, un groupe alcynyle en $C_2$-$C_5$, un groupe alkylthioalkyle en $C_2$-$C_4$ ou un groupe alcoxyalkyle en $C_2$-$C_4$; A représente -O-, -C(O)-, $-OCH_2-$, $-CH_2O-$, $-CH_2S-$, -C≡C-, -CH=CH-, $-CH_2CH_2-$, $-CH_2ON=C(R^4)-$, ou $-CH=NOC(R^4)(R^5)-$ (formules dans lesquelles $R^4$ et $R^5$ représentent indépendamment un atome d'hydrogène ou un groupe alkyle en $C_1$-$C_4$) ; et B représente un atome d'hydrogène, un groupe aryle choisi parmi les groupes phényle et naphtyle ou un groupe hétérocyclique choisi parmi les groupes pyridyle, pyrimidyle, furanyle et benzothiazolyle, lesdits groupes aryle et hétérocycliques étant éventuellement substitués avec un substituant choisi parmi le groupe cyano; le groupe nitro; un atome d'halogène; un groupe alkyle en $C_1$-$C_4$; un groupe haloalkyle en $C_1$-$C_4$; un groupe alcoxy en $C_1$-$C_6$ éventuellement substitué avec un atome d'halogène ou un groupe cycloalkyle en $C_3$-$C_6$; un groupe alkylthio en $C_1$-$C_6$; un groupe alcényloxy en $C_2$-$C_6$ éventuellement substitué avec un atome d'halogène; un groupe alcynyloxy en $C_2$-$C_6$; et un groupe phénoxy, benzyloxy ou pyridyloxy éventuellement substitué avec un groupe cyano, un groupe nitro, un atome d'halogène, un groupe alkyle en $C_1$-$C_4$, un groupe haloalkyle en $C_1$-$C_4$, un groupe alcoxy en $C_1$-$C_6$ éventuellement substitué avec un atome d'halogène ou un groupe cycloalkyle en $C_3$-$C_6$, un groupe alkylthio en $C_1$-$C_6$, un groupe alcényloxy en $C_2$-$C_6$ qui peut être substitué avec un atome d'halogène, ou un groupe alcynyloxy en $C_2$-$C_6$.

**2.** Dérivé de N-pyrazolylcarbamate selon la revendication 1, dans lequel $R^1$ et $R^2$ représentent un groupe alkyle en $C_1$-$C_4$; $R^3$ représente un atome d'hydrogène, un groupe alkyle en $C_1$-$C_4$, un groupe alcynyle en $C_2$-$C_5$ ou un groupe alcoxalkyle en $C_2$-$C_4$; et A représente -O-, $-OCH_2-$, $-CH_2O-$, $-CH_2S-$, -C≡C-, -CH=CH-, $-CH_2ON=C(R^4)-$, ou $-CH=NOC(R^4)(R^5)-$ (formules dans lesquelles $R^4$ et $R^5$ représentent indépendamment un atome d'hydrogène ou un groupe alkyle en $C_1$-$C_4$).

**3.** Dérivé de N-pyrazolylcarbamate selon la revendication 1, dans lequel $R^1$ et $R^2$ représentent un groupe alkyle en $C_1$-$C_4$; $R^3$ représente un atome d'hydrogène, un groupe alkyle en $C_1$-$C_4$, un groupe alcynyle en $C_2$-$C_5$ ou un groupe alcoxalkyle en $C_2$-$C_4$; A représente -O-, $-OCH_2-$, $-CH_2O-$, $-CH_2S-$, -C≡C-, -CH=CH-, $-CH_2ON=C(R^4)-$, ou $-CH=NOC(R^4)(R^5)-$(formules dans lesquelles $R^4$ et $R^5$ représentent indépendamment un atome d'hydrogène ou un groupe alkyle en $C_1$-$C_4$); et B est ledit groupe aryle ou hétérocyclique qui peut contenir un (des) substituant(s)

choisis entre un atome d'halogène, un groupe alkyle en $C_1$-$C_4$, un groupe haloalkyle en $C_1$-$C_4$, un groupe alcoxy en $C_1$-$C_6$ qui peut être substitué avec un atome d'halogène et un groupe phénoxy.

**4.** Dérivé de N-pyrazolylcarbamate selon la revendication 1, dans lequel $R^1$ et $R^2$ représentent un groupe méthyle; $R^3$ représente un atome d'hydrogène, un groupe alkyle en $C_1$-$C_4$, un groupe alcynyle en $C_2$-$C_5$ ou un groupe alcoxyalkyle en $C_2$-$C_4$; A représente $-OCH_2-$, $-CH_2O-$, $-CH_2S-$, $-C{\equiv}C-$, $-CH_2ON{=}C(R^4)-$, ou $-CH{=}NOC(^4)(R^5)-$ (formules dans lesquelles $R^4$ est un atome d'hydrogène ou un groupe méthyle et $R^5$ est un atome d'hydrogène); et B un groupe phényle qui peut contenir un (des) substituant(s) choisis entre un atome d'halogène, un groupe alkyle en $C_1$-$C_4$, un groupe alcoxy en $C_1$-$C_4$ qui peut être substitué par un atome de fluor, un groupe trifluorométhyle et un groupe phénoxy.

**5.** Dérivé de pyrazol-5-ylcarboxylate représenté par la formule générale (II) :

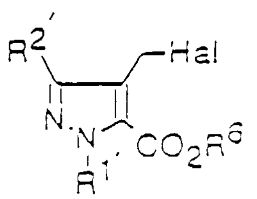 (VIII) ou 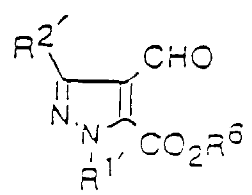 (XIII)

dans laquelle $R^{1'}$ et $R^{2'}$ représentent un groupe alkyle en $C_1$-$C_4$; Hal représente un atome de chlore ou un atome de chlore; et $R^6$ représente un groupe alkyle en $C_1$-$C_4$, en tant qu'intermédiaire pour la production des dérivés de N-pyrazolylcarbamates selon la revendication 2 ou 3.

**6.** Dérivé de 1,3-diméthylpyrazol-5-ylcarboxylate selon la revendication 5, dans lequel $R^{1'}$ et $R^{2'}$ représentent un groupe méthyle.

**7.** Fongicide agricole/horticole comprenant comme ingrédient actif les dérivés de N-pyrazolylcarbamates selon l'une quelconque des revendications 1 - 4.